(19) Europäisches Patentamt
European Patent Office
Office européen des brevets

(11) Numéro de publication: **0 133 603**
**A1**

(12) # DEMANDE DE BREVET EUROPEEN

(21) Numéro de dépôt: 84401637.8

(22) Date de dépôt: 06.08.84

(51) Int. Cl.⁴: C 07 F 5/00
A 61 K 49/00, C 07 F 7/22
C 07 F 9/00, C 07 F 11/00
C 07 F 13/00, C 07 F 15/00

(30) Priorité: 12.08.83 FR 8313281

(43) Date de publication de la demande: 27.02.85 Bulletin 85/9

(84) Etats contractants désignés: BE CH DE FR GB IT LI LU NL SE

(71) Demandeur: COMMISSARIAT A L'ENERGIE ATOMIQUE
Etablissement de Caractère Scientifique Technique et Industriel
31/33, rue de la Fédération
F-75015 Paris(FR)

(72) Inventeur: Bardy, André
48, les Genêts
F-91420 Morangis(FR)

(72) Inventeur: Conti, Maria-Louisa
12, Avenue Porchefontaine
F-78000 Versailles(FR)

(72) Inventeur: Courtieu, Jacques
61, Avenue A. Morizet
F-92100 Boulogne(FR)

(72) Inventeur: Mathieu, Evelyne
5, Villa Gabriel
F-92320 Chatillon(FR)

(72) Inventeur: Grzyb, Joelle
10 Ter, Rue Madame
F-78000 Versailles(FR)

(74) Mandataire: Mongrédien, André et al,
c/o BREVATOME 25, rue de Ponthieu
F-75008 Paris(FR)

(54) **Agents de relaxation spécifiques d'organes ou de pathologies, utilisables pour modifier les contrastes en imagerie médicale par résonance magnétique nucléaire.**

(57) L'invention a pour objet des agents de relaxation spécifiques d'organes ou de pathologies, utilisables pour modifier les contrastes en imagerie médicale par résonance magnétique nucléaire.

Ces agents sont constitués par un complexe d'un élément paramagnétique comportant un ligand biologiquement actif ou un ligand couplé à une molécule biologiquement active, spécifique d'un organe ou d'une pathologie. Ainsi, ils se fixent sélectivement sur les organes et permettent de modifier les contrastes en imagerie par résonance magnétique nucléaire grâce à la présence de l'élément paramagnétique qui modifie les temps de relaxation.

A titre d'exemples, pour l'examen du rein, le complexe peut être constitué par du dimercaptosuccinate ou du diéthylène triaminopentaacétate de gadolinium et, pour l'examen du foie, par le phytate de gadolinium.

EP 0 133 603 A1

Croydon Printing Company Ltd.

# AGENTS DE RELAXATION SPECIFIQUES D'ORGANES OU DE PATHOLOGIES, UTILISABLES POUR MODIFIER LES CONTRASTES EN IMAGERIE MEDICALE PAR RESONANCE MAGNETIQUE NUCLEAIRE

La présente invention a pour objet des agents de relaxation spécifiques d'organes ou de pathologies, utilisables pour modifier les contrastes en imagerie médicale par résonance magnétique nucléaire.

La spectroscopie par résonance magnétique nucléaire (RMN) a été mise au point en 1946 par Bloch et Purcell, et cette technique a été largement utilisée depuis dans les domaines de la physique, de la chimie organique et de la biochimie, pour étudier la structure chimique des molécules et des groupements moléculaires "in vitro". Le principe de la RMN utilise la conjonction de champs magnétiques et d'ondes de radiofréquence pour mettre en résonance les noyaux de certains atomes. L'un des atomes les plus intéressants étant l'hydrogène, qui est présent dans l'eau constituant la majorité des tissus biologiques (75 à 80% des tissus mous), il a été possible d'envisager l'utilisation de la technique RMN dans le domaine médical, et le développement de l'imagerie par résonance magnétique nucléaire dans ce domaine a connu, depuis moins de deux ans, un essor considérable. L'image de la distribution des protons est obtenue à partir du signal émis, après mise en résonance des noyaux, et la reconstruction par ordinateur conduit à la visualisation des coupes transverses, sagittales et frontales du corps humain.

L'analyse des fréquences de résonance ainsi que celle des paramètres caractéristiques des noyaux s'obtient par spectrométrie RMN : c'est l'observation de la réponse impulsionnelle des noyaux à l'excitation par une onde de radiofréquence. Les noyaux reviennent à leur état d'équilibre initial selon des constantes

de temps qui dépendent de leur environnement et sont appelées temps de relaxation.

Le temps de relaxation $T_1$ (dit spin-réseau) caractérise le comportement du noyau observé dans le champ magnétique environnant (modifié par les noyaux et électrons de voisinage). Le temps de relaxation $T_2$ (spin-spin) est significatif des liaisons moléculaires dans lesquelles le noyau observé est impliqué. Sa modification traduit les mouvements microscopiques de ce noyau par rapport aux noyaux voisins.

Un autre paramètre qui peut être mesuré, est la densité $\rho$ des protons dans le milieu ; elle représente en première approximation la quantité d'eau libre contenue dans l'échantillon. Il apparaît donc que la densité de l'eau dans le tissu, mais aussi la mobilité relative des molécules d'eau induisent des variations des paramètres mesurés, ce qui se rencontre dans des conditions pathologiques, mais peut aussi être provoqué par l'introduction d'éléments modifiant le milieu environnant.

Actuellement, l'image par résonance magnétique nucléaire représente la distribution de ces paramètres $\rho$, $T_1$, $T_2$ ou de leur combinaison. Le contraste entre un tissu donné et les tissus adjacents est d'autant plus important que les tissus contiennent plus ou moins d'eau ou de protons mobiles et que les temps de relaxation sont différents. Aussi, on peut modifier le contraste en faisant varier un ou plusieurs de ces paramètres. L'expérience a montré qu'il était plus intéressant de modifier les temps de relaxation pour améliorer le contraste de l'image. En effet, la densité des protons (en pratique ceux de l'eau et des graisses) varie peu d'un organe à l'autre et souvent moins entre tissus normaux et pathologiques. En revanche, les caractéristiques de relaxation dépen-

dent d'un grand nombre de facteurs (dynamique microscopique des molécules, échange chimique, perturbations paramagnétiques...) beaucoup plus variables. Les possibilités techniques de sélection, au moins relative, des différents paramètres pour l'obtention de l'image finale (par expérience d'échos de spins favorisant le rôle de $T_2$, ou par expérience d'inversion-récupération de l'aimentation permettant la mesure locale de $T_1$), ont montré tout l'intérêt de la technique, l'exemple le plus spectaculaire étant celui du constraste possible entre la substance blanche et la substance grise sur les images du cerveau normal ou pathologique.

Malgré l'importance des phénomènes naturels, le contraste peut être encore insuffisant, particulièrement entre tissus sains et pathologiques, au sein d'un même organe, ou pour mettre en évidence des compartiments anatomiques définis tels que les voies biliaires, l'arbre urinaire etc... Aussi, on a envisagé d'accentuer artificiellement le contraste en introduisant dans l'organisme à examiner des éléments modifiant notamment le champ magnétique, par exemple des composés paramagnétiques, afin de perturber les temps de relaxation des noyaux déjà présents dans l'organisme, en ajoutant à la relaxation nucléaire une composante liée à des perturbations par un moment magnétique électronique.

L'utilisation d'agents de contraste de ce type est en particulier décrite par Brasch, Runge et al, et Brasch et al dans Radiology, vol. 147, n°3, juin 1983 pp. 773-791. Ces agents de contraste peuvent être des sels de métaux de transition ou de gadolinium, des radicaux nitroxydes ou de l'éthylènediamine tétraacétate de chrome.

Cependant, l'utilisation de tels agents de

contraste n'est pas satisfaisante, car il est nécessaire d'injecter dans l'organisme une quantité relativement importante de produit pour obtenir l'effet recherché au niveau de l'organe examiné. De ce fait, de nombreux agents ne peuvent être utilisés car ils ne permettent pas l'obtention d'une amélioration significative du signal de RMN, à des doses non toxiques.

La présente invention a précisément pour objet de nouveaux agents de relaxation qui permettent de modifier les contrastes en imagerie médicale par résonance magnétique nucléaire, en palliant l'inconvénient précité.

L'agent de relaxation utilisable pour modifier les contrastes en imagerie médicale par résonance magnétique nucléaire, selon l'invention, se caractérise en ce qu'il est constitué par un complexe d'un élément paramagnétique choisi parmi les lanthanides et les métaux de transition portant les numéros atomiques de 21 à 29, 42 et 44, comportant un ligand biologiquement actif ou un ligand couplé à une molécule biologiquement active, spécifique d'un organe ou d'une pathologie, ledit ligand étant choisi parmi :

1°) les amides répondant à la formule :

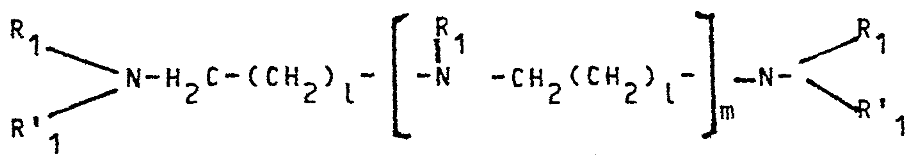

(I)

dans laquelle $R_1$ et $R'_1$ qui peuvent être identiques ou différents, représentent : $CH_2-(CH_2)_l-NH_2$ avec $l$ étant un nombre de 1 à 7, $CH_2-PO_3H_2$, un résidu d'acide aminé, le radical de formule $-R_2-COOH$ avec $R_2$ représentant un radical alcoylène ou alcénylène ayant de 1 à 18 atomes de carbone,

le radical de formule :

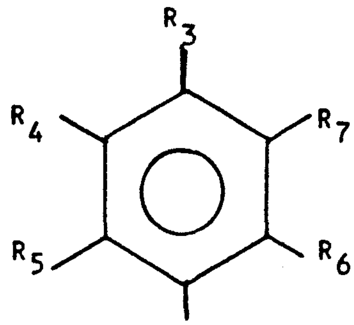

avec $R_3$, $R_4$, $R_5$, $R_6$ et $R_7$ qui peuvent être identiques ou différents, représentant un alkyle en $C_1$ à $C_8$, H, SH, OH, $COOR_8$ avec $R_8$ représentant un atome d'hydrogène ou un métal physiologiquement acceptable, un résidu d'acide aminé, $CH_2PO_3H_2$,

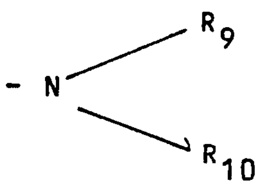 , 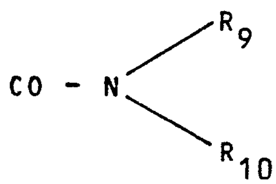

avec $R_9$ et $R_{10}$ qui peuvent être identiques ou différents, représentant H ou un radical alkyle en $C_1$ à $C_4$, et m est un nombre de 0 à 4 ;

2° les composés tétra-aza de formule :

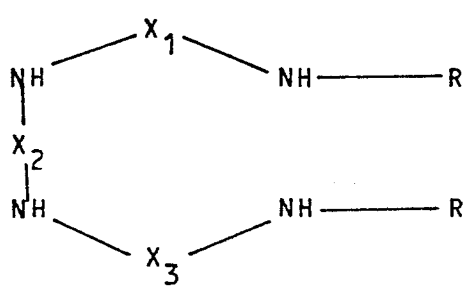 (II)

dans laquelle $X_1$, $X_2$ et $X_3$ qui peuvent être identiques ou différents, représentent des radicaux alcoylène de 2 à 4 atomes de carbone non substitués ou substitués par au moins l'un des groupements suivants : alkyle en $C_1$ à $C_8$, $COOR_8$ avec $R_8$ représentant un atome d'hydrogène ou un métal physiologiquement acceptable, OH, SH, un résidu d'acide aminé, $CH_2PO_3H_2$,

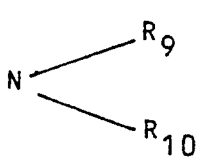 , 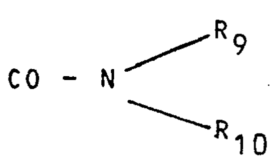

avec $R_9$ et $R_{10}$ représentant l'hydrogène ou un radical alkyle en $C_1$ à $C_4$, et dans laquelle R et R' qui peuvent être identiques ou différents, représentent un atome d'hydrogène, un radical méthyle ou éthyle, ou dans laquelle R et R' forment ensemble un radical alcoylène de 2 à 4 atomes de carbone, non substitué ou substitué par au moins l'un des groupements suivants : alkyle en $C_1$ à $C_8$, $COOR_8$ avec $R_8$ représentant un atome d'hydrogène ou un métal physiologiquement acceptable, OH, SH, un résidu d'acide aminé, $CH_2PO_3H_2$,

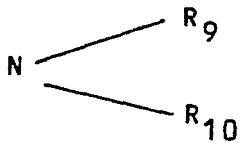 et 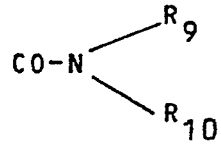

avec $R_9$ et $R_{10}$ représentant l'hydrogène ou un radical alkyle en $C_1$ à $C_4$ ;

3°) les composés tétra-aza de formule :

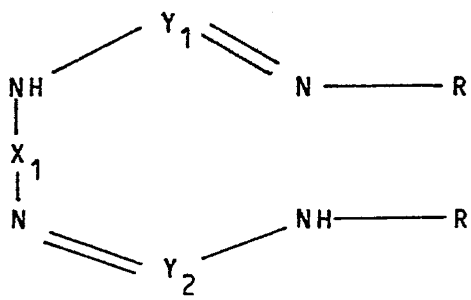

(III)

dans laquelle $X_1$ représente un radical alcoylène de 2 à 4 atomes de carbone, non substitué ou substitué par au moins l'un des groupements suivants :
alkyle en $C_1$ à $C_8$, $COOR_8$ avec $R_8$ représentant un atome d'hydrogène ou un métal physiologiquement acceptable, OH, SH, un résidu d'acide aminé, $CH_2PO_3H_2$,

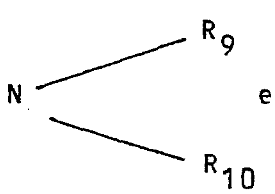 et 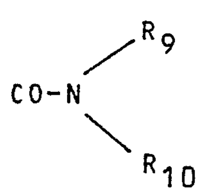

avec $R_9$ et $R_{10}$ représentant l'hydrogène ou un radical alkyle en $C_1$ à $C_4$, $Y_1$ et $Y_2$ qui peuvent être identi-

ques ou différents, représentent un radical trivalent choisi parmi l'éthanyl-1 ylidène-2, le propanyl-1 ylidène-3 et le butanyl-1 ylidène-4, non substitué ou substitué par au moins l'un des groupements suivants : alkyle en $C_1$ à $C_8$, $COOR_8$ avec $R_8$ représentant un atome d'hydrogène ou un métal physiologiquement acceptable, OH, SH, un résidu d'acide aminé, $CH_2PO_3H_2$,

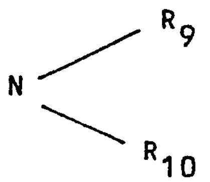 et 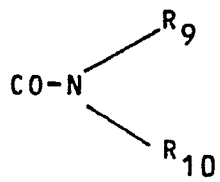

avec $R_9$ et $R_{10}$ représentant l'hydrogène ou un radical alkyle en $C_1$ à $C_4$, et dans laquelle R et R' qui peuvent être identiques ou différents, représentent un atome d'hydrogène, un radical méthyle ou éthyle ou dans laquelle R et R' forment ensemble un radical alcoylène de 2 à 4 atomes de carbone, non substitué ou substitué par au moins l'un des groupements suivants : alkyle en $C_1$ à $C_8$, $COOR_8$ avec $R_8$ représentant un atome d'hydrogène ou un métal physiologiquement acceptable, OH, SH, un résidu d'acide aminé, $CH_2PO_3H_2$,

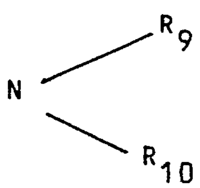 et 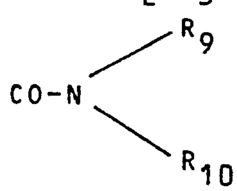

avec $R_9$ et $R_{10}$ représentant l'hydrogène ou un radical alkyle en $C_1$ à $C_4$ ;

4°) les porphyrines répondant à la formule :

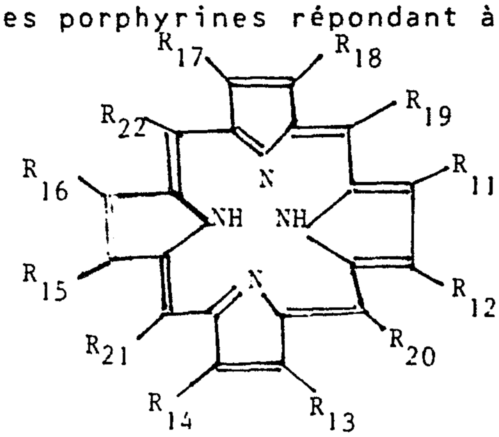

(IV)

dans laquelle $R_{11}$, $R_{12}$, $R_{13}$, $R_{14}$, $R_{15}$, $R_{16}$, $R_{17}$ et $R_{18}$ qui peuvent être identiques ou différents, représentent H, un alkyle en $C_1$ à $C_4$, un alcényle en $C_2$ à $C_4$, $CHOH-R_9$ avec $R_9$ représentant l'hydrogène ou un radical alkyle en $C_1$ à $C_4$, $(CH_2)_n\ COOR_9$, $(CH_2)_n-OH$, $(CH_2)_n-SH$,

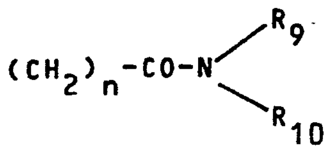

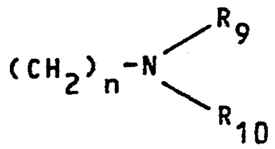

avec n étant un nombre de 1 à 4, $R_9$ et $R_{10}$ qui peuvent être identiques ou différents, représentant un atome d'hydrogène ou un radical alkyle en $C_1$ à $C_4$, et $R_{10}$, $R_{20}$, $R_{21}$ et $R_{22}$ qui peuvent être identiques ou différents, représentent H, un radical phényle, un radical pyridyle ou un radical de formule 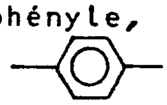 $SO_3R_8$ avec $R_8$ représentant un atome d'hydrogène ou un métal physiologiquement acceptable ;

5°) l'acide myo-inositol hexaphosphorique de formule :

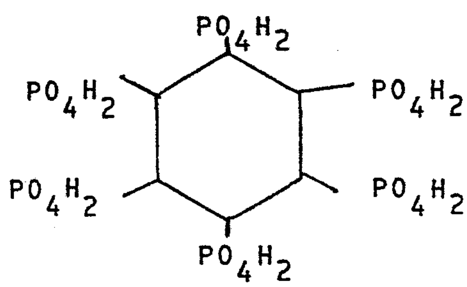

(V)

6°) les dérivés de formule :

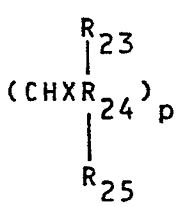

dans laquelle $R_{23}$ et $R_{25}$ qui peuvent être identiques ou différents, représentent $CH_2-COOR_8$, $COOR_8$ avec $R_8$

représentant un atome d'hydrogène ou un métal physiologiquement acceptable, ou $CH_2OH$, X représente O ou S, $R_{24}$ représente H ou $COOR_8$ avec $R_8$ représentant un atome d'hydrogène ou un métal physiologiquement acceptable, et p est un nombre allant de 1 à 6 ;

7°) les phosphines de formule :

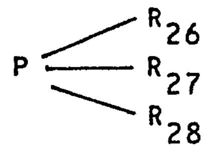

(VII)

dans laquelle $R_{26}$, $R_{27}$ et $R_{28}$ qui peuvent être identiques ou différents, représentent un radical alkyle en $C_1$ à $C_4$, $(CH_2)_q-COOR_8$ avec $R_8$ représentant un atome d'hydrogène ou un métal physiologiquement acceptable, $(CH_2)_qOH$, $(CH_2)_q-PO_3H_2$,

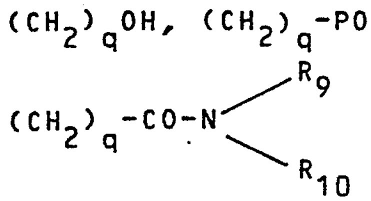

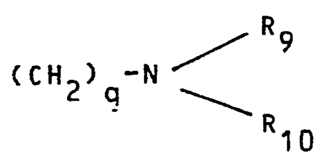

avec q étant un nombre de 1 à 18 et $R_9$ et $R_{10}$ représentant un atome d'hydrogène ou un radical alkyle en $C_1$ à $C_4$ ;

8°) les acides amino polycarboxyliques de formule :

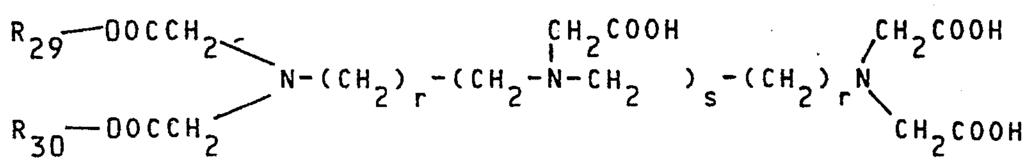

dans laquelle r est un nombre de 1 à 4, s est un nombre de 0 à 2, et $R_{29}$ et $R_{30}$ représentent un atome d'hydrogène ou $R_{29}$ et $R_{30}$ représentent ensemble Ca à condition que $R_{29}$ et $R_{30}$ ne représentent pas H lorsque s est égal à 0 ou 1 et que le ligand n'est pas couplé à une molécule biologiquement active ;

9°) les phénylcarbamoyl-méthyliminodiacétates subs-

titués ou non sur le noyau phényle par des radicaux alkyle en $C_1$ à $C_4$ ;

10°) les bléomycines ;

11°) les bléomycines couplées à un complexant organique ;

12°) les composés diaza macrocycliques de formule :

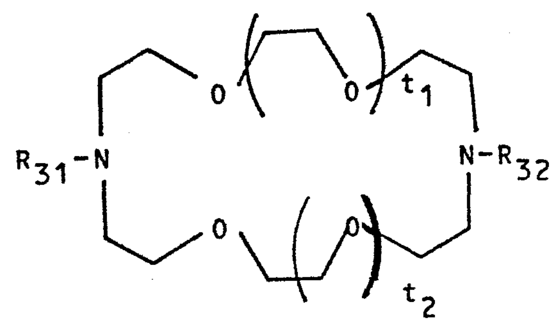

(IX)

dans laquelle $t_1$ et $t_2$ qui sont identiques ou différents, sont des nombres pouvant aller de 0 à 2, et dans laquelle les groupements $R_{31}$ et $R_{32}$ qui peuvent être identiques ou différents, représentent un atome d'hydrogène, $(CH_2)_n-COOR_8$ avec $R_8$ représentant un atome d'hydrogène ou un métal physiologiquement acceptable, $(CH_2)_nSH$, $(CH_2)_nOH$,

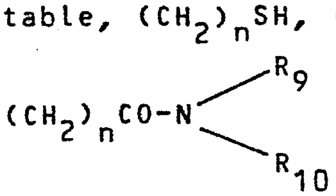 , 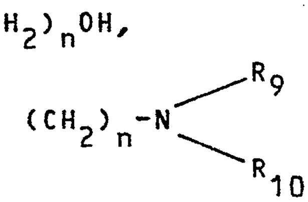

avec $R_9$ et $R_{10}$ qui peuvent être identiques ou différents, représentant un atome d'hydrogène ou un radical alkyle en $C_1$ à $C_4$ et n étant un nombre entier allant de 1 à 4, ou dans laquelle $R_{31}$ et $R_{32}$ peuvent former ensemble le radical $-CH_2-CH_2-O-(CH_2-CH_2-O)_{t_3}-CH_2-CH_2-$ avec $t_3$ étant un nombre de 0 à 2, les différents atomes de carbone du composé diaza pouvant également être substitués par un ou plusieurs radicaux choisis parmi $(CH_2)_n-COOR_8$, $(CH_2)_n-SH$, $(CH_2)_nOH$,

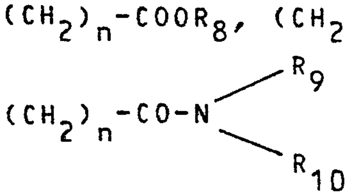 , 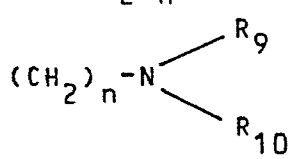

avec $R_9$ et $R_{10}$ qui peuvent être identiques ou différents, représentant un atome d'hydrogène ou un radical alkyle en $C_1$ à $C_4$ et n étant un nombre allant de 1 à 4 ;

13°) l'entérobactine répondant à la formule :

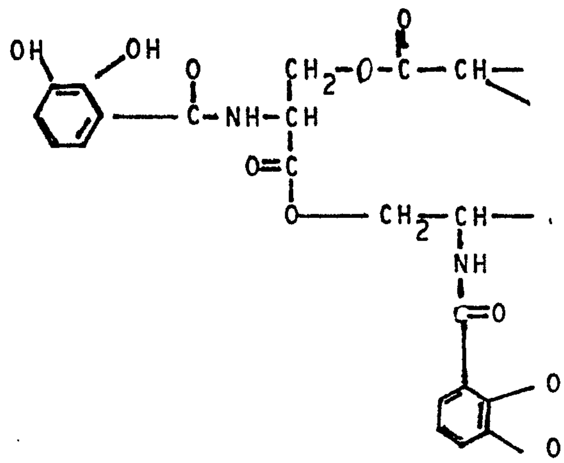

14°) l'analogue carbocyclique de . entérobactine de formule :

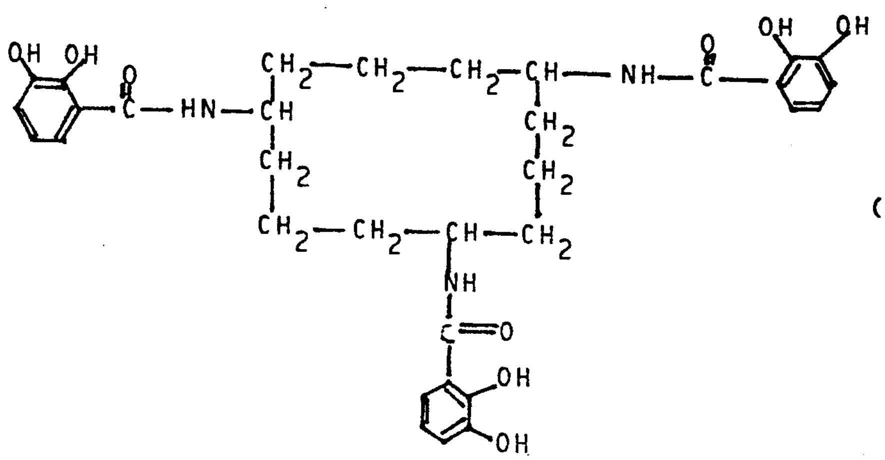

(XI)

15°) le dérivé carbocyclique de formule :

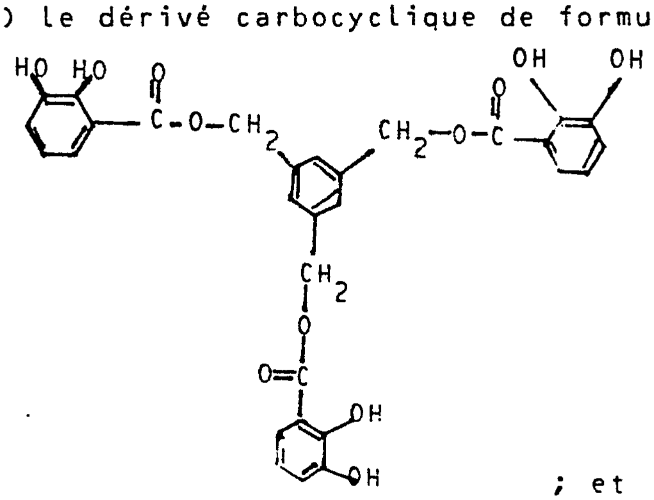

(XII)

; et

16°) les iloxannes de formule :

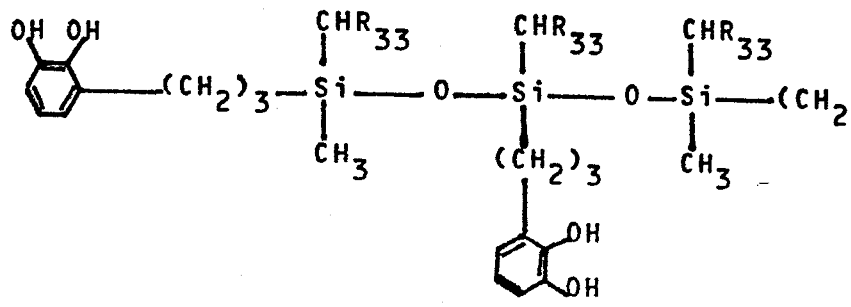

dans laquelle $R_{33}$ représente un atome d'hydrogène, un groupe méthyle ou le groupe $-CH_2-C_6H_4OCH_2COOH$.

On rappelle que dans la série des lanthanides, les éléments suivants sont paramagnétiques : cérium, praséodyme, néodyme, prométhium, samarium, europium, gadolinium, terbium, dysprosium, holmium, erbium et thulium.

De préférence, selon l'invention, le lanthanide paramagnétique est choisi parmi le gadolinium, le dysprosium, l'holmium, le thulium, le terbium et le samarium. Parmi ces derniers éléments, on préfère encore davantage le gadolinium.

Les agents de relaxation de l'invention présentent en particulier l'avantage de pouvoir modifier le contraste d'une image RMN grâce à la présence de l'élément paramagnétique constitué par un lanthanide ou un métal de transition et de pouvoir se fixer sélectivement sur l'organe à examiner en raison du choix du ligand. De ce fait, on peut réduire d'un facteur important la quantité d'agent injectée et atténuer les risques de toxicité. En effet, l'agent de relaxation comporte une partie vectrice constituée par le ligand biologiquement actif, spécifique de l'organe ou de la pathologie à examiner et il se fixe par cette partie

vectrice sur l'organe ou la zone à étudier. Une fois fixé, il améliore le contraste de l'image RMN en raison de la présence de l'élément paramagnétique.

Le fait d'utiliser des complexes de lanthanides paramagnétiques permet d'obtenir le résultat recherché malgré l'union de l'élément paramagnétique à une molécule vectrice. En effet, dans ce cas, l'effet paramagnétique du lanthanide sur les temps de relaxation du proton n'est ni annulé, ni trop diminué par la présence de la molécule ou ligand biologiquement actif. De même, le ligand biologiquement actif conserve ses propriétés, et sa fixation sélective sur l'organe ou la zone à examiner n'est pas perturbé par l'élément paramagnétique.

Selon l'invention, on choisit le ligand du complexe en fonction de l'organe ou de la zone du corps humain à étudier afin d'obtenir une bonne fixation du complexe sur la partie à observer.

Les ligands biologiquement actifs énumérés ci-dessus peuvent comporter différents substituants et le choix de ces substituants permet en particulier de leur conférer la spécificité voulue vis-à-vis de l'organe ou de la pathologie à étudier.

Selon l'invention, on peut en particulier conférer au ligand du complexe la spécificité voulue par couplage de celui-ci avec une molécule biologiquement active choisie par exemple parmi les antigènes, les anticorps, les anticorps monoclonaux, les fragments et combinaisons AC-fragments, les drogues, les récepteurs, les stéroïdes, les aminoacides, les peptides, et les enzymes. On peut utiliser en particulier un anticorps monoclonal. Les anticorps monoclonaux susceptibles d'être utilisés peuvent être obtenus par le procédé décrit par Milstein et Kohler dans Nature 256, p.495-497, 1975. Ce procédé implique pour l'es-

sentiel l'injection d'un immunogène à une souris ou à un autre animal convenable. La souris est ensuite sacrifiée et des cellules prélevées sur sa rate sont unies à des cellules de myélome.

Il en résulte une cellule hybride appelée "hybridome" qui se reproduit in vitro. La population d'hybridomes est sélectionnée et manipulée de manière à isoler des clones individuels dont chacun élabore une espèce unique d'anticorps relative à un antigène. Chaque anticorps spécifique individuel obtenu de cette façon est le produit d'une unique cellule B de l'animal immun, engendrée en réponse à un site antigénique spécifique reconnu sur la substance immunogénique.

Le couplage d'un anticorps monoclonal avec le ligand peut être réalisé par exemple par l'intermédiaire des fonctions acide, amine, phénol, hydroxyle ou cétone de l'anticorps ou du ligand, de préférence par l'intermédiaire d'une fonction amine ou acide, en utilisant les réactions de couplage habituelles et des réactifs tels que les carbodiimides, le glutaraldéhyde, la benzidine, les anhydrides mixtes, les esters actifs de l'anticorps et l'acide paraaminophénylacétique.

Pour réaliser cette réaction, le ligand doit comporter des groupes réactifs tels que :

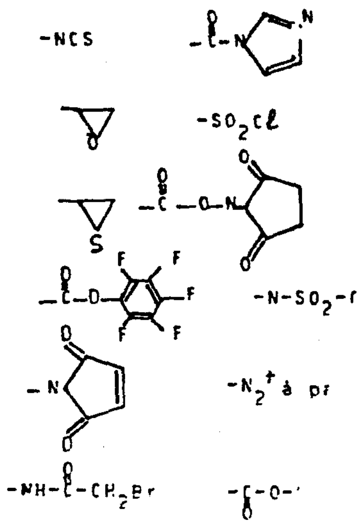

Ceux-ci peuvent être apportés au ligand par des méthodes classiques.

La spécificité biologique peut également être conférée au ligand par l'utilisation de groupements tels que des résidus d'acides aminés, c'est-à-dire des radicaux dérivés d'un acide aminé par enlèvement d'un atome d'hydrogène à un radical alkyle, d'un hydroxyle au groupement COOH ou d'un hydrogène au groupement $NH_2$ de l'acide aminé.

Les acides aminés peuvent être en particulier les acides aminés naturels tels que le glycocolle, l'alanine, la phénylalanine, la leucine, l'asparagine, la glutamine, la lysine, la sérine, la cystine et la tyrosine.

Lorsqu'on veut obtenir une image du cerveau ou étudier la pathologie du cerveau, on peut utiliser des complexes constitués par des gluconates, des dicitrates ou des polycitrates de lanthanides paramagnétiques ou du diéthylène triaminopentaacétate de calcium et de lanthanide paramagnétique. Pour les complexes constitués par les gluconates et les dicitrates, le ligand répond à la formule VI avec $R_{23}$ et $R_{25}$ représentant $CH_2-COOH$, X représentant O, $R_{24}$ représentant

COOH et p = 1 dans le cas du citrate. Pour le diéthylènetriaminopentacétate de calcium et de lanthanide, le ligand répond à la formule VIII avec r et s égaux à 1. Ces complexes peuvent être également obtenus par action de l'acide correspondant sur un sel ou un oxyde du lanthanide paramagnétique.

Pour l'étude du coeur, on peut utiliser des complexes comportant comme ligands des trialkyl phosphines de formule (VII).

Lorsqu'on veut obtenir une image du rein, on utilise avantageusement des complexes constitués par des gluconates, des dimercaptosuccinates ou des thiomalates de lanthanides paramagnétiques, du diéthylènetriaminopentacétate de calcium et de lanthanide paramagnétique ou du triéthylène tétraminohexacétate de lanthanide. Ces complexes sont également obtenus par réaction de l'acide correspondant avec un sel ou un oxyde du lanthanide paramagnétique.

Lorsqu'on veut obtenir une image des voies biliaires, on utilise avantageusement un complexe constitué par un phénylcarbamoyl-méthyliminodiacétate de lanthanide paramagnétique substitué ou non sur le noyau phényle.

Pour l'étude du foie, on peut utiliser un phytate de lanthanide paramagnétique, soit un myo-inositol hexaphosphate de lanthanide de formule (V).

Pour l'étude et la détection des tumeurs, on peut utiliser des complexes de lanthanides dans lesquels le ligand biologiquement actif est une porphyrine ou une bléomycine.

Les porphyrines ou dérivés de la porphine ont la structure générale :

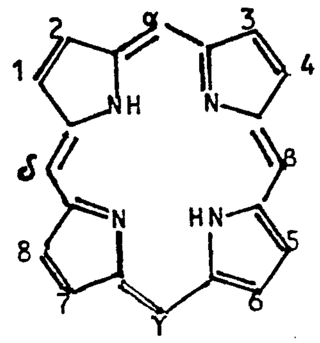

On peut obtenir plusieurs dérivés hydrosolubles en effectuant des substitutions en alpha, bêta, gamma et/ou delta.

A titre d'exemple de dérivé hydrosoluble, on peut citer la tétraphényl porphyrine sulfonée, c'est-à-dire la porphyrine de formule (IV) dans laquelle $R_{19}$, $R_{20}$, $R_{21}$ et $R_{22}$ représentent le radical :

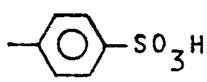

et dans laquelle $R_{11}$, $R_{12}$, $R_{13}$, $R_{14}$, $R_{15}$, $R_{16}$, $R_{17}$ et $R_{18}$ représentent un atome d'hydrogène.

Par complexation de ces dérivés hydrosolubles par un lanthanide paramagnétique, on obtient des agents ayant une forte affinité tumorale.

Les bléomycines sont des molécules plus importantes qui peuvent complexer les lanthanides et qui ont une spécificité pour une pathologie car elles présentent également une affinité pour les tumeurs.

Les bléomycines peuvent complexer directement les lanthanides paramagnétiques, mais on peut aussi obtenir un agent de relaxation en couplant tout d'abord une bléomycine avec un complexant organique ayant plusieurs sites de complexations tels qu'un acide aminopolycarboxylique comme l'acide diéthylènetriaminopentacétique, l'acide triéthylène tétraminohexacétique ou l'acide éthylènediaminotétracétique, et en complexant ensuite le lanthanide paramagnétique

au moyen du complexant couplé à la bléomycine.

On donne, ci-après, la formule de la bléomycine :

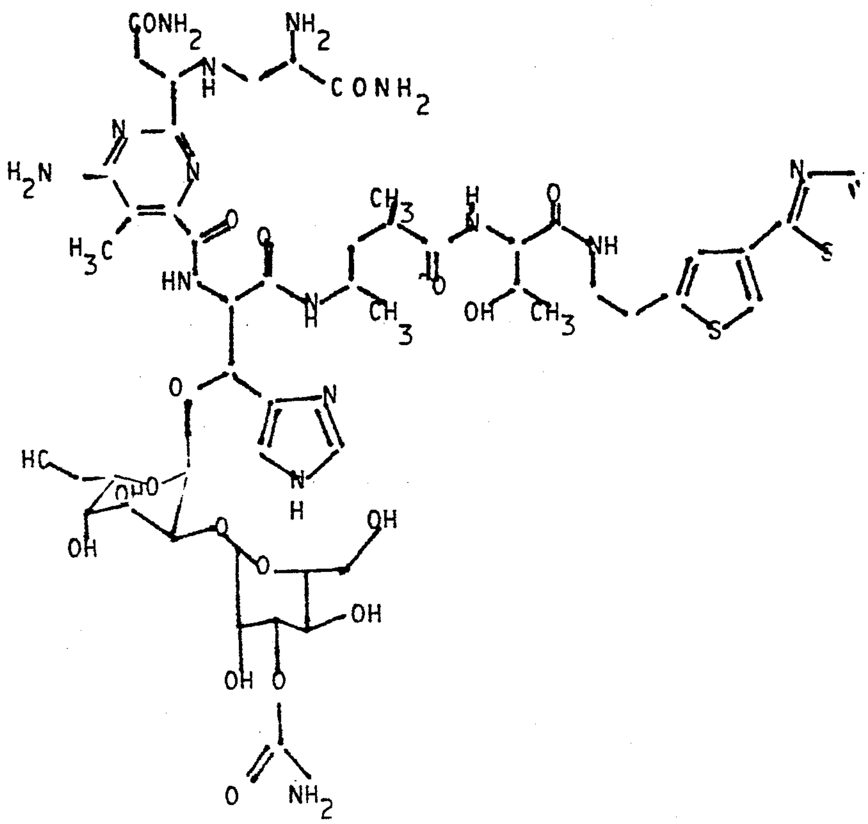

A titre d'exemples, Z peut représenter :

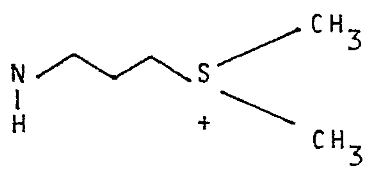 (bléomycine $A_2$)

ou 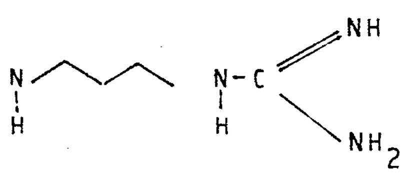 (bléomycine $B_2$)

Généralement, on utilise les complexes de lanthanide paramagnétique de l'invention sous la forme de solutions stériles, apyrogènes, par exemple sous la forme de solution dans du sérum physiologique, et on les injecte par voie intraveineuse, par voie intrarachidienne ou encore par voie orale. Les doses injectées sont généralement faibles et sont choisies compte tenu de la toxicité du produit, de façon à obtenir une modification optimale du contraste sans engendrer de toxicité.

Ainsi, dans le cas du dimercaptosuccinate de gadolinium la dose injectée peut correspondre à 0,6 µmol de gadolinium et 1,8 µmol de dimercaptosuccinate pour 200 g de poids du corps. Ceci est très en dessous de la dose pour laquelle apparaît un effet de toxicité aiguë dû au gadolinium, qui est de 29,6 µmol de gadolinium pour 200 g, soit de 55 mg de Gd par kg. Par ailleurs, la dose injectée est choisie en fonction du taux de fixation du complexe sur l'organe à examiner et de la grandeur de cet organe, tout en tenant compte de la toxicité.

Après injection du produit, on attend que le taux de fixation de ce produit sur l'organe à examiner ait atteint son maximum. Ce maximum peut varier en fonction de la nature du complexe utilisé. Ainsi, dans le cas du phytate de gadolinium, le taux de fixation du complexe sur le foie est d'environ 80% et ce taux est atteint au bout de 5 à 30 minutes environ. Dans le cas du dimercaptosuccinate de gadolinium, on obtient un taux de fixation maximal de 15% sur le rein et ce taux est atteint en environ 2 h.

Dès qu'on a atteint ce taux maximal de fixation, on peut réaliser une image de l'organe à examiner par résonance magnétique nucléaire en utilisant des appareillages classiques.

On peut encore améliorer la spécificité des agents de relaxation de l'invention, en les conditionnant sous une forme appropriée. Ainsi, on peut les utiliser sous la forme de colloïdes en suspension dans une solution aqueuse, ce qui permet de les fixer préférentiellement sur les glandes du système lymphatique ou dans le système hépatique en fonction de la granulométrie du colloïde. Ainsi, des complexes tels que les citrates de lanthanides conditionnés sous forme colloïdale, par exemple d'une taille de particules d'environ 10 nm, en présence de stabilisants tels que la polyvinylpyrrolidone ou la gélatine, peuvent être utilisés dans une forme galénique appropriée à la lymphographie indirecte par injection sous-cutanée de la suspension.

On peut aussi inclure les complexes de lanthanides de l'invention dans des liposomes et obtenir ainsi des agents de relaxation ayant une affinité plus importante pour le foie, la rate et certains tissus.

Ceci peut être effectué par des procédés classiques tels que ceux décrits dans Labo-Pharma-Problèmes et Techniques, n°281, novembre 1978, p.905 à 907 ; Presse Médicale, 12 mai 1979, 8, n°21, p.1749 à 1752 et Journal Français de Biophysique et Médecine Nucléaire, 1, 1979, 3, (1), p.3 à 12.

Les exemples suivants sont donnés à titre non limitatif pour illustrer l'invention.

EXEMPLE 1

Cet exemple concerne la préparation du dimercaptosuccinate de gadolinium et son utilisation pour obtenir une image du rein par résonance magnétique nucléaire.

On dissout dans de l'eau additionnée de quelques gouttes de soude, l'acide dimercaptosuccinique en s'assurant que le pH ne dépasse pas 2,5. On

ajoute ensuite à la solution une solution de chlorure de gadolinium de façon à obtenir un rapport molaire acide dimercaptosuccinique/$Gd^{3+}$ de 3/1, une concentration en acide dimercaptosuccinique de la solution finale au plus égale à 6 $mmol.l^{-1}$ et une concentration en gadolinium au plus égale à 2 $mmol.l^{-1}$. On ajuste le pH à 2,5 et on conserve le produit à l'abri de la lumière à 4°C dans une atmosphère inerte.

On a vérifié que le produit obtenu donne un élargissement du pic du proton inversement proportionnel au temps de relaxation $T_2$ de 4 Hz en solution à 1$mmol.l^{-1}$ (la largeur du pic du proton de l'eau est d'environ 1 Hz).

Ainsi, l'effet du dimercaptosuccinate de gadolinium est pratiquement identique à celui de l'ion $Gd^{3+}$ qui donne un élargissement de 4,5 Hz en solution à 1 $mmol.l^{-1}$.

On injecte à des rats de 200 g, 0,2 $cm^3$ de la solution obtenue, ce qui correspond à une dose de 0,6 µmol de gadolinium et 1,8 µmol d'acide dimercaptosuccinique. Après 90 minutes, les rats sont sacrifiés, et leurs reins prélevés. On examine alors les reins prélevés par RMN, en utilisant l'appareil BRUKER WP 60 dont le spectre RMN par transformée de Fourier en présence d'un gradient de champ statique sur z fournit une projection en tranches de l'organe étudié.

Dans ces conditions, on obtient une variation du temps de relaxation $T_1$ atteignant localement 50% et globalement 15% pour le rein entier. Par ailleurs, on a vérifié que le dimercaptosuccinate de gadolinium se fixe à 30% sur les deux reins.

<u>EXEMPLE 2</u>

Cet exemple concerne la préparation de phytate de gadolinium.

On prépare une solution à 20 $mmol.l^{-1}$ d'aci-

de phytique en traitant une solution de phytate de sodium par une résine cationique AG 50 x 8 ayant une dimension de particules inférieures à 0,038 mm ( 400 mesh), et on obtient ainsi une solution dont le pH est de 1,8. On dissout du chlorure de gadolinium hexahydraté dans de l'eau distillée et on ajoute à cette solution, une quantité d'acide phytique telle que le rapport molaire $Gd^{3+}$/ acide phytique soit de 1 :6. On ajuste alors le pH à 4,8 et on dilue la solution pour obtenir la concentration désirée qui est généralement inférieure à 3 $mmol.l^{-1}$.

On a vérifié que ce produit donne un élargissement du pic du proton de l'eau inversement proportionnel au temps de relaxation $T_2$ de 16 Hz pour une solution à 1 $mmol.l^{-1}$.

L'effet de ce complexe est donc supérieur à celui du gadolinium ionique (4,5 Hz en solution à 1 $mmol.l^{-1}$).

Ce produit est utilisé pour l'étude du foie car il se fixe sur le foie à des taux supérieurs à 80%.

Après avoir injecté par voie intraveineuse à des rats des doses de 0,0017 mmol de phytate de gadolinium et avoir attendu environ 20 à 30 min, les rats ont été sacrifiés et les foies prélevés après dissection. La mesure in vitro du $T_s$ des morceaux de foie des rats a montré que l'injection de phytate de gadolinium a provoqué une diminution du $T_s$ d'environ 17%.

EXEMPLE 3

Cet exemple concerne la préparation du diéthylène triamino pentaacétate de gadolinium et de calcium. On met en suspension dans 60 ml d'eau, 2,5 g de $Gd_2/_3$ et 5 g d'acide diéthylènetriaminopentaacétique. On agite la suspension pendant environ 48 h à environ 50°C. L'acide diéthylènediaminopentaacétique dissout lentement l'oxyde de gadolinium. On dilue à

100 ml et on ajoute de l'acétone pour précipiter le complexe de gadolinium formé. On filtre à l'abri de l'humidité et on sèche le précipité sous vide dans un dessicateur avec du chlorure de calcium.

Le précipité ainsi obtenu est dissous à nouveau dans de l'eau distillée et traité à chaud (environ 80°C) avec une suspension de carbonate de calcium tout en agitant pendant 24 h. On filtre le carbonate he calcium en excès et on précipite par un excès d'acétone le sel mixte de diéthylènetriaminopentaacétate de gadolinium et calcium, puis on sèche le précipité sous vide.

La mesure de la $DL_{50}$ de ce produit sur 6 groupes de 5 souris mâles et sur 6 groupes de 5 souris femelles a donné les résultats suivants :

$DL_{50}$ = 900 $\pm$ 82,7 mg/kg pour les souris mâles,
$DL_{50}$ = 1158 $\pm$ 92,3 mg/kg pour les souris femelles,
$DL_0$ = 479 mg/kg

Ceci correspond donc à une $DL_{50}$ de 1,65 mmol/kg. Le sel mixte est soluble dans l'eau et le pH est de 6,8.

Le produit obtenu donne un élargissement du pic du proton de l'eau inversement proportionnel au temps de relaxation $T_2$ de 3,4 Hz en solution à 1 mmol.$l^{-1}$.

Ce produit est utilisable pour l'étude des anomalies de perfusion des organes tels que le rein, le cerveau, etc...

EXEMPLE 4

Cet exemple concerne la préparation d'un complexe de gadolinium d'une amine macrocyclique.

On dissout séparément dans du méthanol des quantités équimolaires de $GdCl_3$ et de l'amine macrocyclique constituée par le "Cyclam" soit le 1,4,8,11 tétraazacyclodécane. On mélange 2 mmol de $GdCl_3$ anhydre

dissous dans le méthanol avec 2 mmol de "Cyclam" dissous aussi dans le méthanol en mélangeant les deux solutions et en les portant au reflux pendant environ 3 h. On filtre la solution et on précipite le complexe avec de l'éther, puis on sèche le précipité sous vide. La mesure du temps de relaxation $T_1$ pour des solutions allant de 5 mmol à 0,2 mmol donne un abaissement par rapport à l'eau de 1/20 à 1/4.

EXEMPLE 5

Cet exemple concerne la préparation d'un complexe de gadolinium marqué par l'anticorps monoclonal commercialisé sous la référence 19.9.

Cet anticorps monoclonal est spécifique des cancers colorectaux humains et on a utilisé le fragment Fab'$_2$ de l'anticorps pour réaliser cette synthèse.

On couple 20 mg d'anticorps, soit $2.10^{-7}$ mol, en solution dans du bicarbonate de soude 0,1M pH8 avec $40.10^{-7}$ mol de DTPA (diéthylène triamino pentacétate) anhydride en solution dans du diméthyl sulfoxide. Ce qui représente un rapport molaire DTPA/anticorps de 20. Le couplage se fait en 15 min sous agitation à la température ambiante.

On purifie l'anticorps couplé au DTPA par filtration sur une colonne (12 cmX2,5 cm) de Séphadex G100. La quantité d'anticorps couplée au DTPA est déterminée après mesure de la densité optique à 280 nm. On sait que dans ces conditions de couplage, on a couplé 8 molécules de DTPA/molécule d'anticorps, soit pour $2.10^{-7}$ mol d'anticorps, $16\ 10^{-7}$ mol de DTPA.

La complexation du gadolinium à l'anticorps couplé au DTPA se fait de la manière suivante.

On ajoute $16.10^{-7}$ mol de chlorure de podolinium en solution dans de l'eau distillée à la solution contenant l'anticorps couplé au DTPA.

Le rapport gadolinium/DTPA est donc de 1. La complexation a lieu en 1h sous agitation à la température ambiante.

On purifie le complexe gadolinium-anticorps formé par l'intermédiaire du DTPA par filtration sur une colonne (10 cmx1,5 cm) de Séphadex G100.

Le complexe gradolinium anticorps via le DTPA est récupéré et après voir déterminé la quantité d'anticorps complexé au gadolinium par la mesure de la densité optique à 280 nm, on conserve le complexe à +4°C.

On a donc obtenu un complexe gadolinium-DTPA-anticorps, dont on connaît la concentration en anticorps (20 mg/ml) et en gadolinium ($10^6$ mol/ml). Ce produit donne un élargissement du pic du proton de l'eau inversement proportionnel au temps de relaxation $T_2$ de 2,9 Hz en solution à 1 $mmol.l^{-1}$.

<u>EXEMPLE 6</u>

Préparation de cryptates de gadolinium dans lesquels le ligand répond aux formules suivantes :

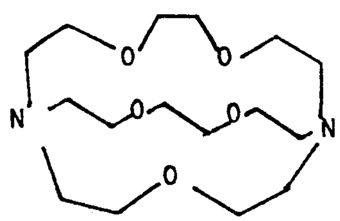

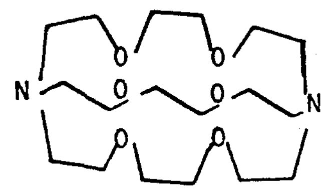

On dissout 0,5 mmol de cryptand et 0,5 mmol de chlorure de gadolinium anhydre dans 60 ml de méthanol anhydre. On fait bouillir à reflux pendant 4h de façon anhydre sous atmosphère inerte. On évapore ensuite le volume du solvant à environ 40 ml et on précipite le complexe avec de l'éther sec. On filtre et on sèche le produit sous vide. Ces complexes de gado-

linium donnent un élargissement du pic du proton inversement proportionnel au temps de relaxation $T_2$, de 2,8 Hz pour une solution à 1 mmol par litre.

EXEMPLE 7

Cet exemple concerne la préparation de triéthylènetétraminohexacétate de gadolinium.

On mélange des quantités équimoléculaires d'oxyde de gadolinium et d'acide triéthylènetétraminohexacétique dans de l'eau et on uhauffe à environ 90°C jusqu'à dissolution. On refroidit et on précipite le complexe avec de l'acétone en excès. On filtre et on sèche le produit sous vide. Ce produit qui contient 2 atomes de gadolinium par molécule présente un élargissement du pic du proton, inversement proportionnel au temps de relaxation $T_2$, de 8 Hz pour une solution à 1 mmol par litre.

## REVENDICATIONS

1. Agent de relaxation utilisable pour modifier les contrastes en imagerie médicale par résonance magnétique nucléaire, caractérisé en ce qu'il est constitué par un complexe d'un élément paramagnétique choisi parmi les lanthanides et les métaux de transition portant les numéros atomiques de 21 à 29, 42 et 44, comportant un ligand biologiquement actif ou un ligand couplé à une molécule biologiquement active, spécifique d'un organe ou d'une pathologie, ledit ligand étant choisi parmi :

1°) les amines répondant à la formule :

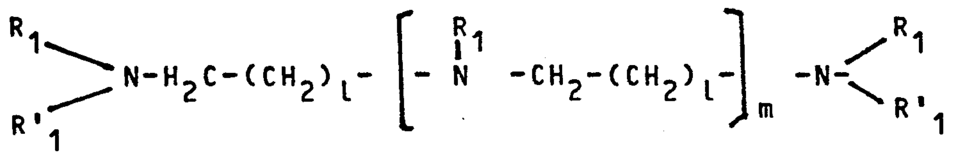

(I)

dans laquelle $R_1$ et $R'_1$ qui peuvent être identiques ou différents, représentent : $CH_2-(CH_2)_l-NH_2$ avec $l$ étant un nombre de 1 à 7, $CH_2-PO_3H_2$, un résidu d'acide aminé, le radical de formule $-R_2-COOH$ avec $R_2$ représentant un radical alcoylène ou alcénylène ayant de 1 à 18 atomes de carbone,

le radical de formule :

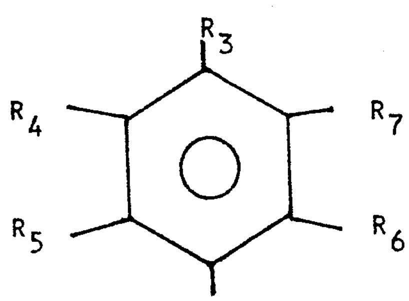

avec $R_3$, $R_4$, $R_5$, $R_6$ et $R_7$ qui peuvent être identiques ou différents, représentant un alkyle en $C_1$ à $C_8$, H, SH, OH, $COOR_8$ avec $R_8$ représentant un atome d'hydrogène ou un métal physiologiquement acceptable, un résidu d'acide aminé, $CH_2PO_3H_2$,

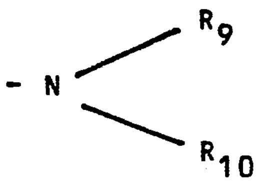 , 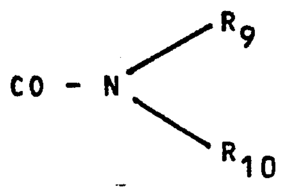

avec $R_9$ et $R_{10}$ qui peuvent être identiques ou différents, représentant H ou un radical alkyle en $C_1$ à $C_4$, et m est un nombre de 0 à 4 ;

2° les composés tétra-aza de formule :

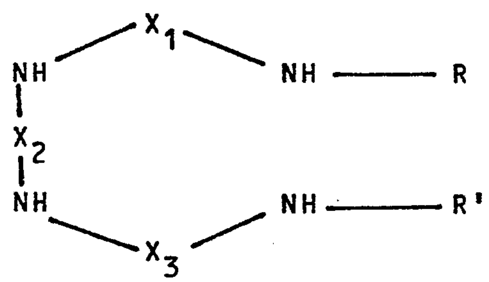 (II)

dans laquelle $X_1$, $X_2$ et $X_3$ qui peuvent être identiques ou différents, représentent des radicaux alcoylène de 2 à 4 atomes de carbone non substitués ou substitués par au moins l'un des groupements suivants : alkyle en $C_1$ à $C_8$, $COOR_8$ avec $R_8$ représentant un atome d'hydrogène ou un métal physiologiquement acceptable, OH, SH, un résidu d'acide aminé, $CH_2PO_3H_2$,

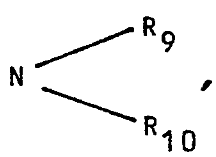 , 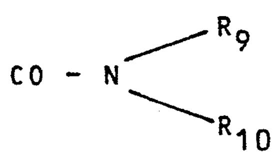

avec $R_9$ et $R_{10}$ représentant l'hydrogène ou un radical alkyle en $C_1$ à $C_4$, et dans laquelle R et R' qui peuvent être identiques ou différents, représentent un atome d'hydrogène, un radical méthyle ou éthyle, ou dans laquelle R et R' forment ensemble un radical alcoylène de 2 à 4 atomes de carbone, non substitué ou substitué par au moins l'un des groupements suivants : alkyle en $C_1$ à $C_8$, $COOR_8$ avec $R_8$ représentant un atome d'hydrogène ou un métal physiologiquement acceptable, OH, SH, un résidu d'acide aminé, $CH_2PO_3H_2$,

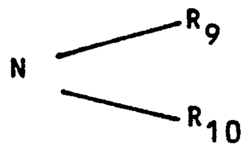 et 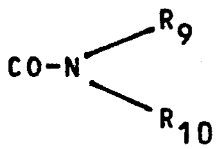

avec $R_9$ et $R_{10}$ représentant l'hydrogène ou un radical alkyle en $C_1$ à $C_4$ ;

3°) les composés tétra-aza de formule :

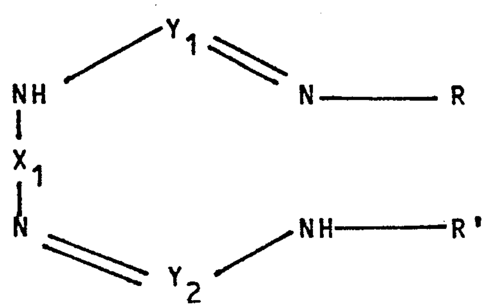

(III)

dans laquelle $X_1$ représente un radical alcoylène de 2 à 4 atomes de carbone, non substitué ou substitué par au moins l'un des groupements suivants :
alkyle en $C_1$ à $C_8$, $COOR_8$ avec $R_8$ représentant un atome d'hydrogène ou un métal physiologiquement acceptable, OH, SH, un résidu d'acide aminé, $CH_2PO_3H_2$,

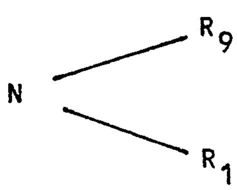 et 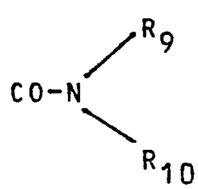

avec $R_9$ et $R_{10}$ représentant l'hydrogène ou un radical alkyle en $C_1$ à $C_4$, $Y_1$ et $Y_2$ qui peuvent être identiques ou différents, représentent un radical trivalent choisi parmi l'éthanyl-1 ylidène-2, le propanyl-1 ylidène-3 et le butanyl-1 ylidène-4, non substitué ou substitué par au moins l'un des groupements suivants :
alkyle en $C_1$ à $C_8$, $COOR_8$ avec $R_8$ représentant un atome d'hydrogène ou un métal physiologiquement acceptable, OH, SH, un résidu d'acide aminé, $CH_2PO_3H_2$,

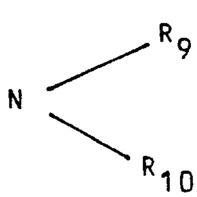 et 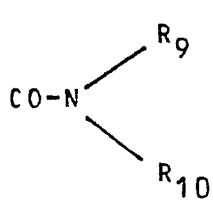

avec $R_9$ et $R_{10}$ représentant l'hydrogène ou un radical alkyle en $C_1$ à $C_4$, et dans laquelle R et R' qui peuvent être identiques ou différents, représentent un atome d'hydrogène, un radicalméthyle ou éthyle ou dans laquelle R et R' forment ensemble un radical alcoylène de 2 à 4 atomes de carbone, non substitué ou substitué par au moins l'un des groupements suivants : alkyle en $C_1$ à $C_8$, $COOR_8$ avec $R_8$ représentant un atome d'hydrogène ou un métal physiologiquement acceptable, OH, SH, un résidu d'acide aminé, $CH_2PO_3H_2$,

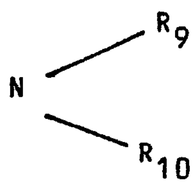 et 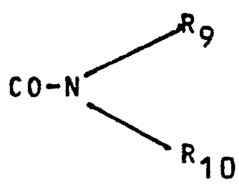

avec $R_9$ et $R_{10}$ représentant l'hydrogène ou un radical alkyle en $C_1$ à $C_4$ ;

4°) les porphyrines répondant à la formule :

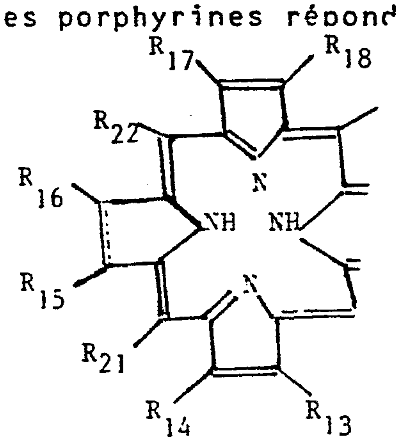

dans laquelle $R_{11}$, $R_{12}$, $R_{13}$, $R_{14}$, $R_{15}$, $R_{16}$, $R_{17}$ et $R_{18}$ qui peuvent être identiques ou différents, représentent H, un alkyle en $C_1$ à $C_4$, un alcényle en $C_2$ à $C_4$, $CHOH-R_9$ avec $R_9$ représentant l'hydrogène ou un radical alkyle en $C_1$ à $C_4$, $(CH_2)_n\ COOR_9$, $(CH_2)_n-OH$, $(CH_2)_n-SH$,

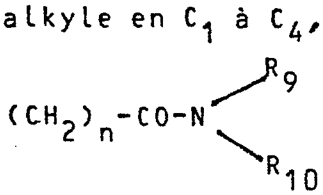

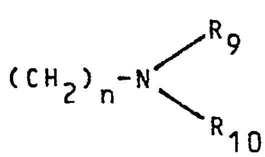

avec n étant un nombre de 1 à 4, $R_9$ et $R_{10}$ qui peuvent être identiques ou différents, représentant un atome d'hydrogène ou un radical alkyle en $C_1$ à $C_4$, et $R_{19}$, $R_{20}$, $R_{21}$ et $R_{22}$ qui peuvent être identiques ou différents, représentent H un radical phényle, un radical pyridyle ou un radical de formule $SO_3R_8$ avec $R_8$ représentant un atome d'hydrogène ou un métal physiologiquement acceptable ;

5°) l'acide myo-inositol hexaphosphorique de formule :

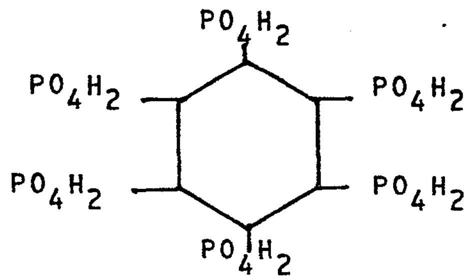

(V)

6°) les dérivés de formule :

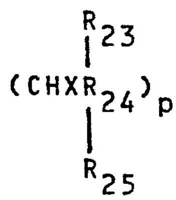

dans laquelle $R_{23}$ et $R_{25}$ qui peuvent être identiques ou différents, représentent $CH_2$-$COOR_8$, $COOR_8$ avec $R_8$ représentant un atome d'hydrogène ou un métal physiologiquement acceptable, $CH_2OH$, X représente O ou S, $R_{24}$ représente H ou $COOR_8$ avec $R_8$ représentant un atome d'hydrogène ou un métal physiologiquement acceptable, et p est un nombre allant de 1 à 6 ;

7°) les phosphines de formule :

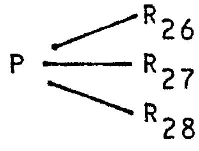

(VII)

dans laquelle $R_{26}$, $R_{27}$ et $R_{28}$ qui peuvent être identiques ou différents, représentent un radical alkyle en

$C_1$ à $C_4$, $(CH_2)_q-COOR_8$ avec $R_8$ représentant un atome d'hydrogène ou un métal physiologiquement acceptable, $(CH_2)_qOH$, $(CH_2)_q-PO_3H_2$,

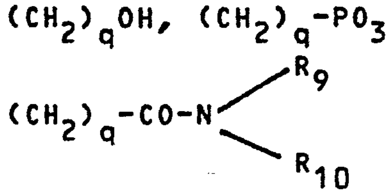

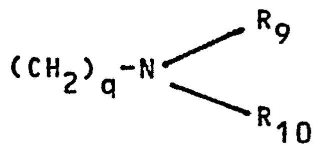

avec q étant un nombre de 1 à 18 et $R_9$ et $R_{10}$ représentant un atome d'hydrogène ou un radical alkyle en $C_1$ à $C_4$ ;

8°) les acides amino polycarboxyliques de formule :

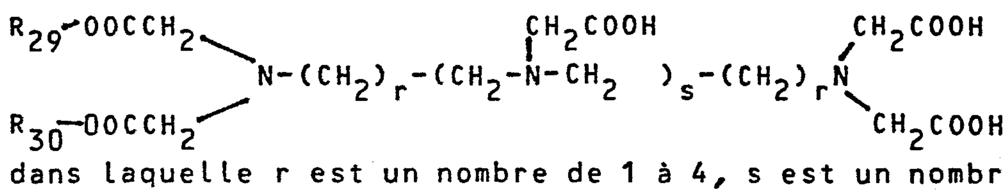 (VIII)

dans laquelle r est un nombre de 1 à 4, s est un nombre de 0 à 2, et $R_{29}$ et $R_{30}$ représentent un atome d'hydrogène ou $R_{29}$ et $R_{30}$ représentent ensemble Ca à condition que $R_{29}$ et $R_{30}$ ne représentent pas H lorsque s est égal à 0 ou 1 et que le ligand n'est pas couplé à une molécule biologiquement active ;

9°) les phénylcarbamoyl-méthyliminodiacétates substitués ou non sur le noyau phényle par des radicaux alkyle en $C_1$ à $C_4$ ;

10°) les bléomycines ;

11°) les bléomycines couplées à un complexant organique ;

12°) les composés diaza macrocycliques de formule :

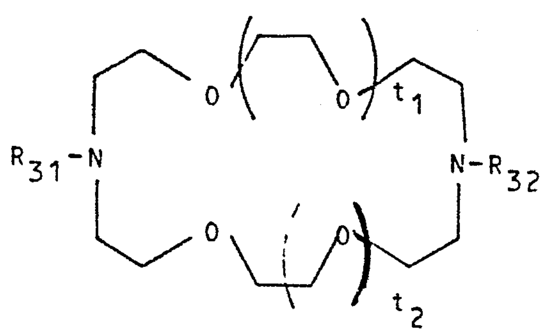 (IX)

dans laquelle $t_1$ et $t_2$ qui sont identiques ou différents, sont des nombres pouvant aller de 0 à 2, et dans laquelle les groupements $R_{31}$ et $R_{32}$ qui peuvent être identiques ou différents, représentent un atome d'hydrogène, $(CH_2)_n-COOR_8$ avec $R_8$ représentant un atome d'hydrogène ou un métal physiologiquement acceptable, $(CH_2)_nSH$, $(CH_2)_nOH$,

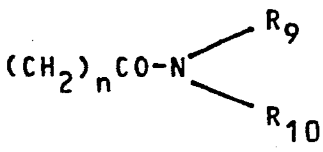 , 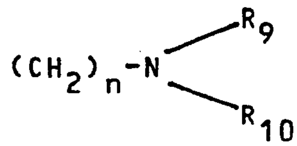

avec $R_9$ et $R_{10}$ qui peuvent être identiques ou différents, représentant un atome d'hydrogène ou un radical alkyle en $C_1$ à $C_4$ et n étant un nombre entier allant de 1 à 4, ou dans laquelle $R_{31}$ et $R_{32}$ peuvent former ensemble le radical $-CH_2-CH_2-O-(CH_2-CH_2-O)_{t_3}-CH_2-CH_2-$ avec $t_3$ étant un nombre de 0 à 2, les différents atomes de carbone du composé diaza pouvant également être substitués par un ou plusieurs radicaux choisis parmi $(CH_2)_n-COOR_8$, $(CH_2)_n-SH$, $(CH_2)_nOH$,

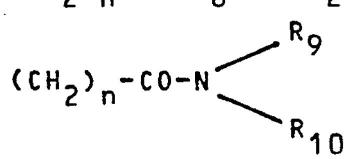 , 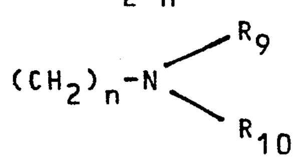

avec $R_9$ et $R_{10}$ qui peuvent être identiques ou différents, représentant un atome d'hydrogène ou un radical alkyle en $C_1$ à $C_4$ et n étant un nombre allant de 1 à 4 ;

13°) l'entérobactine répondant à la formule :

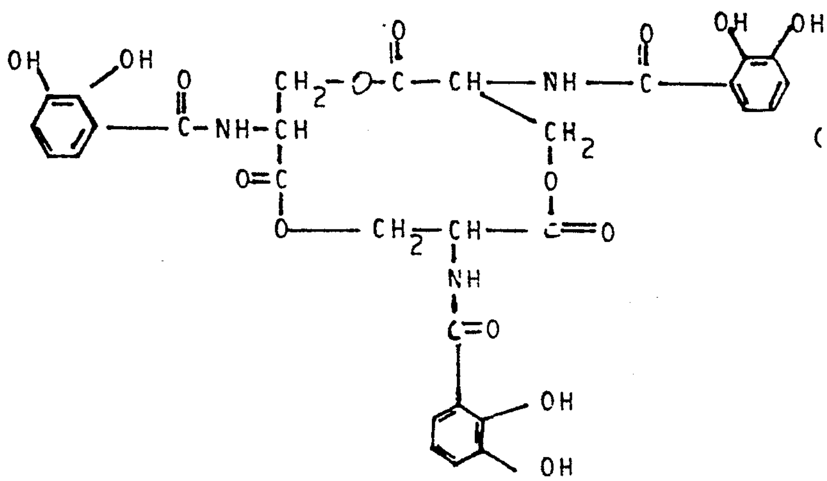

(X)

14°) l'analogue carbocyclique de l'entérobactine de formule :

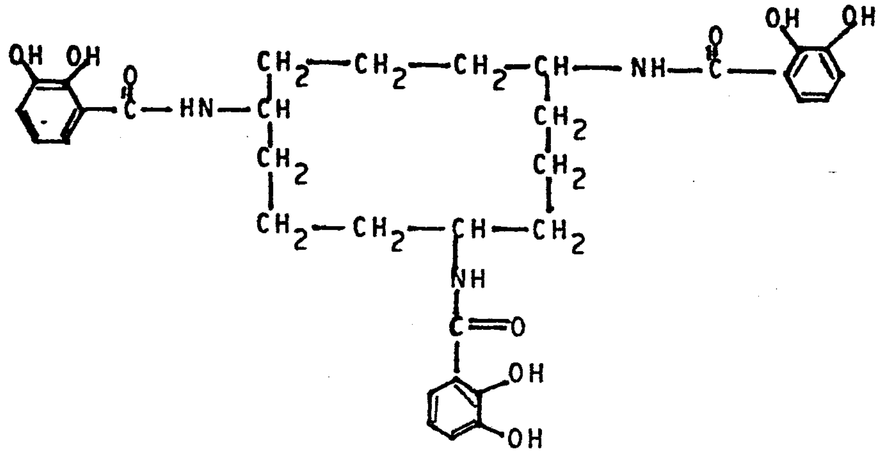

(XI)

15°) le dérivé carbocyclique de formule :

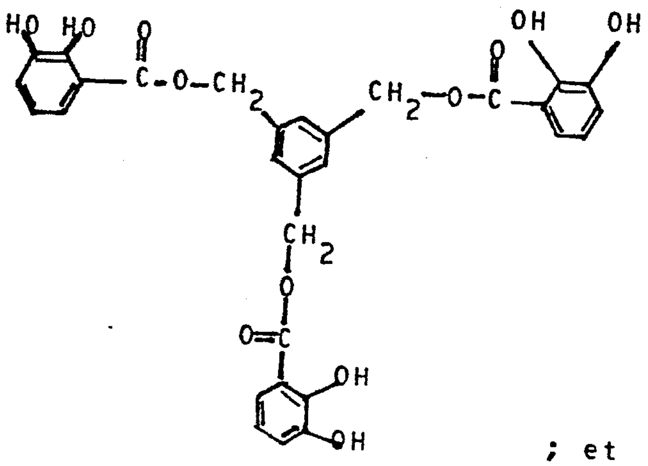

(XII)

; et

16°) les siloxannes de formule :

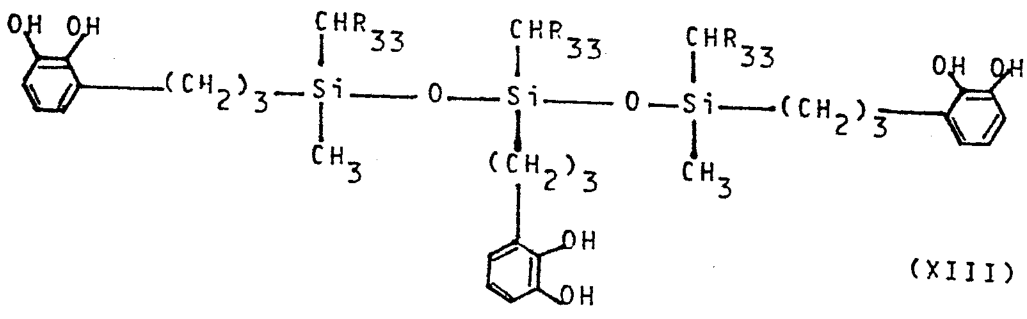

(XIII)

dans laquelle $R_{33}$ représente un atome d'hydrogène, un

groupe méthyle ou le groupe $-CH_2-C_6H_4OCH_2COOH$.

2. Agent selon la revendication 1, caractérisé en ce que le lanthanide est choisi parmi le gadolinium, le dysprosium, l'holmium, le thulium, le terbium et le samarium.

3. Agent selon la revendication 1, caractérisé en ce que l'élément paramagnétique est le gadolinium.

4. Agent de relaxation pour l'examen du rein selon l'une quelconque des revendications 1 à 3, caractérisé en ce qu'il est constitué par un dimercaptosuccinate de lanthanide paramagnétique.

5. Agent de relaxation pour l'examen du foie selon l'une quelconque des revendications 1 à 3, caractérisé en ce qu'il est constitué par un phytate (myo-inositol hexaphosphate) de lanthanide paramagnétique.

6. Agent de relaxation pour l'examen du rein selon l'une quelconque des revendications 1 à 3, caractérisé en ce qu'il est constitué par un diéthylène triamino pentaacétate de lanthanide paramagnétique et de calcium.

7. Agent de relaxation pour l'examen du cerveau selon l'une quelconque des revendications 1 à 3, caractérisé en ce qu'il est constitué par un gluconate, un dicitrate, un polycitrate de lanthanide paramagntique ou un diéthylène triaminopentacétate de calcium et de lanthanide paramagnétique.

8. Agent de relaxation pour l'examen du rein selon l'une quelconque des revendications 1 â 3, caractérisé en ce qu'il est constitué par un gluconate de lanthanide paramagnétique.

9. Agent de relaxation pour l'examen des voies biliaires selon l'une quelconque des revendications 1 à 3, caractérisé en ce qu'il est constitué par

un phénylcarbamoyl-méthyliminodiacétate de lanthanide paramagnétique substitué ou non sur le noyau phényle par des radicaux alkyle en $C_1$ à $C_4$.

10. Agent de relaxation selon la revendication 1, caractérisé en ce que le ligand est couplé à un anticorps monoclonal spécifique d'une pathologie.

11. Agent de relaxation spécifique d'une pathologie selon la revendication 10, caractérisé en ce qu'il est constitué par le diéthylènetriaminopentacétate de gadolinium couplé à un anticorps monoclonal.

12. Agent de relaxation spécifique d'une pathologie selon la revendication 1, caractérisé en ce que le ligand organique est constitué par une bléomycine couplée à un complexant organique constitué par un acide aminopolycarboxylique.

13. Agent de relaxation selon la revendication 12, caractérisé en ce que l'acide aminopolycarboxylique est l'acide diéthylènetétraminopentacétique, l'acide éthylènediaminotétracétique ou l'acide triéthylènetétraminohexacétique.

14. Agent de relaxation selon l'une quelconque des revendications 1 à 3, caractérisé en ce que le ligand est un cryptate.

15. Agent de relaxation selon l'une quelconque des revendications 1 à 3, caractérisé en ce que le ligand est l'acide triéthylènetétraminohexacétique.

16. Agent de relaxation pour l'examen de la fonction hépatique ou du système lymphatique selon l'une quelconque des revendications 1 à 15, caractérisé en ce que le complexe de l'élément paramagnétique est sous la forme d'un colloïde en suspension dans une solution aqueuse.

17. Agent de relaxation selon l'une quelconque des revendications 1 à 15, caractérisé en ce que

le complexe de l'élément paramagnétique est inclus dans des liposomes.

0133603

Office européen des brevets

# RAPPORT DE RECHERCHE EUROPEENNE

Numéro de la demande

EP 84 40 1637

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | EP-A-0 071 564 (SCHERING AG)<br>* Revendications *<br>----- | 1-17 | C 07 F 5/00<br>A 61 K 49/00<br>C 07 F 7/22<br>C 07 F 9/00<br>C 07 F 11/00<br>C 07 F 13/00<br>C 07 F 15/00 |
| | | | **DOMAINES TECHNIQUES RECHERCHES (Int. Cl.4)** |
| | | | C 07 F 5/00<br>A 61 K 49/00 |

Le present rapport de recherche a été établi pour toutes les revendications

| Lieu de la recherche | Date d'achèvement de la recherche | Examinateur |
|---|---|---|
| LA HAYE | 01-11-1984 | SUTER M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant